(19) Europäisches Patentamt
European Patent Office
Office européen des brevets

(11) EP 4 122 325 A1

(12) # EUROPEAN PATENT APPLICATION

(43) Date of publication:
**25.01.2023 Bulletin 2023/04**

(21) Application number: **22186936.5**

(22) Date of filing: **27.03.2013**

(51) International Patent Classification (IPC):
***A23L 2/58** (2006.01)* ***A23L 5/44** (2016.01)*
***A61K 31/015** (2006.01)* ***A61K 31/11** (2006.01)*

(52) Cooperative Patent Classification (CPC):
(C-Sets available)
**A23L 2/58; A23L 5/44; A61K 31/015; A61K 31/11;**
A23V 2002/00 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2012 EP 12162549**

(62) Document number(s) of the earlier application(s) in accordance with Art. 76 EPC:
**13712787.4 / 2 830 443**

(27) Previously filed application:
**27.03.2013 PCT/EP2013/056553**

(71) Applicant: **DSM IP Assets B.V.**
**6411 TE Heerlen (NL)**

(72) Inventors:
- **BADOLATO BÖNISCH, Gabriela**
**4303 KAISERAUGST (CH)**
- **SCHAFFNER, David**
**4303 KAISERAUGST (CH)**
- **ZWICK, Thomas**
**4303 KAISERAUGST (CH)**
- **HUNZIKER, André**
**4303 KAISERAUGST (CH)**

(74) Representative: **DSM Intellectual Property**
**P.O. Box 4**
**6100 AA Echt (NL)**

Remarks:
This application was filed on 26-07-2022 as a divisional application to the application mentioned under INID code 62.

(54) **CLEAR LIQUID CAROTENOID FORMULATIONS AND CLEAR BEVARAGES CONTAINING THEM**

(57) The present invention is directed to a clear liquid formulation comprising:
a) at least one carotenoid, and
b) at least one modified food starch, and
c) at least one saccharide, and
d) water,
especially to a clear liquid formulation comprising
a) 0.1 to 10 weight-% (preferably 0.5 to 5.0 weight-%, more preferably 0.5 to 3.0 weight%, most preferably 1.0 to 3.0 weight-%) of at least one carotenoid, and
b) 20 to 60 weight-% (preferably 30 to 50 weight-%) of at least one modified food starch, and
c) 0.5 to 60 weight-% (preferably 0.5 to 30 weight-%, more preferably 0.5 to 20 weight-%, most preferably 1.0 to 10 weight-%) of at least one saccharide, and
d) 35 to 75 weight-% (preferably 45 to 65 weight-%) of water,
all amounts based on the total weight of the liquid formulation,
whereby all amounts add up to 100 weight-%.

The present invention is further directed to a process for the manufacture of such liquid formulations, as well as to beverages containing them. These beverages are also clear and color stable.

EP 4 122 325 A1

(52) Cooperative Patent Classification (CPC): (Cont.)

C-Sets
A23V 2002/00, A23V 2200/02, A23V 2200/044,
A23V 2250/1878, A23V 2250/211, A23V 2250/61

## Description

The present invention relates to clear, stable, liquid carotenoid formulations, which - when used in a liquid, especially in a beverage such as a soft drink, allows to obtain transparent liquids (even after pasteurization and also at low pH), i.e. clear beverages.

Carotenoids can be used as colorants. The carotenoids can be obtained from a natural source, by fermentation or by chemical synthesis.

The use of carotenoids in beverages is well known. But to provide liquid aqueous formulations comprising at least one carotenoid, at least one modified food starch and at least one saccharide, which allows to prepare a clear (non-turbid, non-opaque) and pasteurization-stable liquid (especially beverages, such as soft drinks) is not yet achieved in a sufficient manner.

Usually after the pasteurization step such beverages are getting very turbid, so that they are not transparent anymore. For the use in many beverages (i.e. soft drinks) a transparent form is desired for marketing reasons.

Therefore the goal of the present invention was to find a liquid formulation, which does not have the above mentioned disadvantages. A further object of the present invention is to replace azo dyes in beverages. Thus, in the liquid formulations of the present invention no azo dye as e.g. disclosed in US 2010/0028444 is present.

The usual process for producing beverages (i.e. soft drinks) is that a carotenoid is emulsified in a first step. Afterwards this emulsion is incorporated into a beverage.

Surprisingly, it has been found that when a carotenoid is emulsified with certain amounts of at least one modified food starch and certain amounts of at least one saccharide so that the carotenoid is embedded in a matrix of this modified food starch and the saccharide a liquid formulation is obtained, which can be used in (aqueous) liquids, which are then pasteurization-stable and transparent (after pasteurization and also at low pH); i.e. are suitable for the manufacture of clear beverages, especially suitable for the manufacture of clear soft drinks. This is especially surprising since only these two matrix components (modified food starch and saccharide) are necessary to achieve the desired result.

Therefore the present invention relates to a clear liquid formulation comprising

a) at least one carotenoid, and
b) at least one modified food starch, and
c) at least one saccharide, and
d) water.

Hereby the carotenoid is embedded in a matrix of this modified food starch and the saccharide.

The expression "at least one" means in the case of compound a) e.g. that only one carotenoid, but also a mixture of two or more carotenoids may be present. The same applies accordingly to compound b) and c).

The term "clear liquid formulation" in the context of the present invention means that if the liquid formulation according to the present invention is diluted with deionized water so that the concentration of the carotenoid is 10 ppm, the initial turbidity is ≤ 30 NTU, preferably the initial turbidity is ≤ 25 NTU, more preferably the initial turbidity is ≤ 20 NTU, most preferably the initial turbidity is ≤ 15 NTU. This especially applies if the carotenoid is β-carotene or 8'-apo-β-caroten-8'-al.

The term "clear beverage" in the context of the present invention means that if the liquid formulation according to the present invention is added to a beverage so that the concentration of the carotenoid in the beverage is 10 ppm, the initial turbidity is ≤ 30 NTU, preferably the initial turbidity is ≤ 25 NTU, more preferably the initial turbidity is ≤ 20 NTU, most preferably the initial turbidity is ≤ 15 NTU. Advantageously the turbidity of the beverage after a storage time of 3 months is ≤ 50 NTU. This especially applies if the carotenoid is β-carotene or 8'-apo-β-caroten-8'-al and the beverage is a soft drink as prepared according to the example given below.

In a preferred embodiment of the present invention the clear liquid formulation according to the present invention is used in soft drinks which generally have a pH in the range of 2.5 to 5.0. Such soft drinks which contain the clear liquid formulation according to the present invention in such a concentration so that the concentration of the carotenoid in the soft drink is 10 ppm show an initial turbidity of ≤ 30 NTU. Preferably the initial turbidity is ≤ 25 NTU, more preferably the initial turbidity is ≤ 20 NTU, most preferably the initial turbidity is ≤ 15 NTU. Advantageously the turbidity of the soft drink after a storage time of 3 months is ≤ 50 NTU. This especially applies if the carotenoid is β-carotene or 8'-apo-β-caroten-8'-al.

The present invention especially relates to a clear liquid formulation comprising:

a) 0.1 to 10 weight-% (preferably 0.5 to 5 weight-%, more preferably 0.5 to 3.0 weight-%, most preferably 1.0 to 3.0 weight-%) of at least one carotenoid, and
b) 20 to 60 weight-% (preferably 30 to 50 weight-%) of at least one modified food starch, and
c) 0.5 to 60 weight-% (preferably 0.5 to 30 weight-%, more preferably 0.5 to 20 weight-%, even more preferably 0.5

to 10 weight-%, most preferably 1.0 to 10 weight-%) of at least one saccharide, and
d) 35 to 75 weight-% (preferably 45 to 65 weight-%) of water,

all amounts based on the total weight of the liquid formulation,
whereby all amounts add up to 100 weight-%.

Hereby the carotenoid/s is/are embedded in a matrix of the modified food starch/es and the saccharide/s.

In a preferred embodiment of the liquid formulation according to the present invention only the compounds a) to d), especially in the amounts given above, are present. That means that the present invention preferably relates to a clear liquid formulation consisting of

a) at least one carotenoid, and
b) at least one modified food starch, and
c) at least one saccharide, and
d) water.

Thus, preferably no other compounds are present. Such not-being-present compounds are the following ones:

- Gum Acacia; Gum Arabic; also modified as disclosed in WO 2008/110225;
- Gum Ghatti as e.g. described in WO 2009/147158;
- Proteins such as gelatin (fish, swine, bovine gelatin);
- Plant proteins;
- Milk proteins;
- Ligninsulfonate;
- Conjugates of plant gums and modified food starch, especially those as disclosed in WO 2011/039336;
- Sodium lauryl sulfate and other sodium alkyl sulfates.

This preference applies for all liquid formulations of the present invention.

Detailed description

The liquid formulation according to the present invention is now described in more detail.

Compound a) - Carotenoid

The term "carotenoid" as used herein comprises a natural or synthetic carotene or structurally related polyene compound which can be used as a functional health ingredient or colorant for food, such as α-carotene, β-carotene, 8'-apo-β-carotenal (8'-apo-β-caroten-8'-al), 8'-apo- β-carotenoic alkyl acid esters such as the ethyl ester (ethyl-8'-apo-β-caroten-8'-oate), canthaxanthin, astaxanthin, astaxanthin (di)esters such as astaxanthin disuccinate, lycopene, lutein, zeaxanthin or crocetin, or mixtures thereof. The preferred carotenoids are β-carotene, 8'-apo-β-carotenal and mixtures thereof, especially β-carotene or 8'-apo-β-carotenal alone, most preferably 8'-apo-β-carotenal.

Therefore a preferred embodiment of the present invention relates to a liquid formulation as described above, wherein at least one carotenoid is chosen from the group consisting of α-carotene, β-carotene, 8'-apo-β-carotenal (8'-apo-β-caroten-8'-al), 8'-apo-β-carotenoic acid esters such as the ethyl ester (ethyl-8'-apo-β-caroten-8'-oate), canthaxanthin, astaxanthin, astaxanthin (di)esters such as astaxanthin disuccinate, lycopene, lutein, zeaxanthin and crocetin, preferably β-carotene and 8'-apo-β-carotenal, most preferably 8'-apo-β-carotenal.

Compound b) - "Modified food starch"

A modified food starch is a food starch that has been chemically modified by known methods to have a chemical structure which provides it with a hydrophilic and a lipophilic portion. Preferably the modified food starch has a long hydrocarbon chain as part of its structure (preferably C5-C18).

At least one modified food starch is preferably used to make a liquid formulation of this invention, but it is possible to use a mixture of two or more different modified food starches in one liquid formulation.

Starches are hydrophilic and therefore do not have emulsifying capacities. However, modified food starches are made from starches substituted by known chemical methods with hydrophobic moieties. For example starch may be treated with cyclic dicarboxylic acid anhydrides such as succinic anhydrides, substituted with a hydrocarbon chain (see O. B. Wurzburg (editor), "Modified Starches: Properties and Uses, CRC Press, Inc. Boca Raton, Florida, 1986, and

subsequent editions). A particularly preferred modified food starch of this invention has the following formula (I)

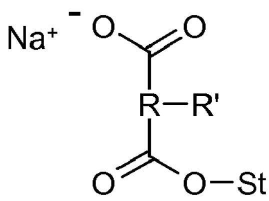

(I)

wherein St is a starch, R is an alkylene radical and R' is a hydrophobic group. Preferably R is a lower alkylene radical such as dimethylene or trimethylene. R' may be an alkyl or alkenyl group, preferably having 5 to 18 carbon atoms. A preferred compound of formula (I) is an "OSA-starch" (starch sodium octenyl succinate). The degree of substitution, i.e. the number of esterified hydroxyl groups to the number of free non-esterified hydroxyl groups usually varies in a range of from 0.1% to 10%, preferably in a range of from 0.5% to 4%, more preferably in a range of from 3% to 4%.

The term "OSA-starch" denotes any starch (from any natural source such as corn, waxy maize, waxy corn, wheat, tapioca and potato or synthesized) that was treated with octenyl succinic anhydride (OSA). The degree of substitution, i.e. the number of hydroxyl groups esterified with OSA to the number of free non-esterified hydroxyl groups usually varies in a range of from 0.1% to 10%, preferably in a range of from 0.5% to 4%, more preferably in a range of from 3% to 4%. OSA-starches are also known under the expression "modified food starch".

The term "OSA-starches" encompasses also such starches that are commercially available e.g. from National Starch under the tradenames HiCap 100, Capsul (octenylbutanedioate amylodextrin), Capsul HS, Purity Gum 2000, Clear Gum Co03, UNI-PURE, HYLON VII; from National Starch and Roquette Frères, respectively; from CereStar under the tradename C*EmCap or from Tate & Lyle.

In a preferred embodiment of the present invention a commercially available modified food starch such as e.g. HiCap 100 (from National Starch) and ClearGum Co03 (from Roquette Frères) is used. It is especially advantageous if such a starch or an OSA starch in general is further improved according to a process as disclosed in WO 2007/090614, especially according to a procedure as described in examples 28, 35 and/or 36 of WO 2007/090614.

Thus, in a further improved embodiment of the present invention such a commercially available starch has been centrifuged as an aqueous solution or suspension before use. The centrifugation may be carried out at 1000 to 20000 g depending on the dry mass content of the modified polysaccharide in the aqueous solution or suspension. If the dry mass content of the modified polysaccharide in the aqueous solution or suspension is high, the applied centrifugation force is also high. For example for an aqueous solution or suspension with a dry mass content of the modified polysaccharide of 30 weight-% a centrifugation force of 12000 g may be suitable to achieve the desired separation.

The centrifugation may be carried out at dry matter contents in the range of from 0.1-60 weight %, preferably in the range of from 10-50 weight-%, most preferably in the range of from 15-40 weight-% at temperatures in the range of from 2-99°C, preferably in the range of from 10-75°C, most preferably in the range of from 40-60°C.

Compound c) - "saccharide"

The term "saccharide" in the context of the present invention encompasses mono-, di-, oligo- and polysaccharides, as well as any mixtures thereof.

Examples of monosaccharides are fructose, glucose (= dextrose), mannose, galactose, sorbose, as well as any mixtures thereof.

Preferred monosaccharides are glucose and fructose, as well as any mixture thereof.

The term "glucose" in the context of the present invention does not only mean the pure substance, but also a glucose syrup with a DE $\geq$ 90. This also applies for the other monosaccharides.

The term "dextrose equivalent" (DE) denotes the degree of hydrolysis and is a measure of the amount of reducing sugar calculated as D-glucose based on dry weight; the scale is based on native starch having a DE close to 0 and glucose having a DE of 100.

Examples of disaccharides are saccharose, isomaltose, lactose, maltose and nigerose, as well as any mixture thereof.

An example of an oligosaccharide is maltodextrin.

An example of a polysaccharide is dextrin.

An example of a mixture of mono- and disaccharides is invert sugar (glucose + fructose + saccharose).

Mixtures of mono- and polysaccharides are e.g. commercially available under the tradenames Glucidex IT 47 (from Roquette Frères), Dextrose Monohydrate ST (from Roquette Frères), Sirodex 331 (from Tate & Lyle) and Glucamyl F 452 (from Tate & Lyle).

In an embodiment of the present invention the saccharide c) is a mixture of a glucose syrup with a DE of 95 and a glucose syrup with a DE of 47 in a weight ratio of 1 : 1.
Such mixtures of glucose syrups are preferred examples of the saccharide c).

Amounts of compounds a) to d)

Amount of compound a)

A liquid formulation according to the present invention comprises preferably 0.1 to 10 weight-%, more preferably 0.5 to 5.0 weight-%, even more preferably 0.5 to 3.0 weight-%, most preferably 1.0 to 3.0 weight-%, of at least one carotenoid based on the total weight of the liquid formulation, whereby the most preferred carotenoids are β-carotene and 8'-apo-β-carotenal.

Amount of compound b)

A liquid formulation according to the present invention comprises preferably 20 to 60 weight-%, more preferably 30 to 50 weight-%, of modified food starch based on the total weight of the liquid formulation, whereby the preferred modified food starch is commercially available OSA-starch, which is preferably further improved by separating off insoluble parts as disclosed e.g. in WO 2007/090614 (examples for centrifugation and microfiltration). If a mixture of two or more modified food starches is present the total amount is also in the ranges as given above.

Amount of compound c)

A liquid formulation according to the present invention comprises preferably 0.5 to 60 weight-%, more preferably 0.5 to 30 weight-%, even more preferably 0.5 to 20 weight-% and 0.5 to 20 weight-%, most preferably 1.0 to 10 weight-%, of a saccharide based on the total weight of the liquid formulation.
In case the saccharide c) is a mixture of glucose and a glucose syrup with a DE $\leq$ 60 the amount of glucose is preferably in the range of 0 to 30 weight-%, more preferably in the range of 0 to 10 weight-%, based on the total weight of the liquid formulation, and/or (preferably and) the amount of the glucose syrup with a DE $\leq$ 60 is preferably in the range of 1 to 30 weight-%, more preferably in the range of 0.5 to 10 weight-%, based on the total weight of the liquid formulation.

Amount of compound d)

A liquid formulation according to the present invention comprises preferably 35 to 75 weight-%, more preferably 45 to 65 weight-%, of water, based on the total weight of the liquid formulation.
In the most preferred embodiment of the present invention all preferred amounts for each compound a) to d) are realized.
If, however, additional compounds are present as e.g. compounds e) and/or compounds f) and/or compounds g) as described below, the amount of water is reduced accordingly.
In preferred embodiments of the liquid formulations of the present invention no other compounds than compounds a) to g) are present, whereby compounds e), f) and g) are independently from each other optional. That means that preferred embodiments of the liquid formulations of the present invention are the following:

- A liquid formulation consisting only of compounds a) to d);
- A liquid formulation consisting only of compounds a) to e);
- A liquid formulation consisting only of compounds a) to f);
- A liquid formulation consisting only of compounds a) to g).

In further preferred embodiments of the liquid formulations of the present invention **none** of the following compounds is present:

- Gum Arabic; Gum Acacia; Gum Ghatti;
- Any protein such as gelatin (fish, swine, bovine);
- Any plant protein;
- Any milk protein;
- Ligninsulfonate;
- Plant gum and modified food starch conjugates as e.g. disclosed in WO 2011/039336;

- No emulsifier (sodium lauryl sulfate).

Furthermore, such clear liquid formulations according to the present invention are preferred, wherein the modified food starch b) has been centrifuged before use.

Advantageously the particle size of the inner phase of the clear liquid formulation is in the range of 100 to 250 nm, preferably in the range of 110 to 210 nm, more preferably in the range of 130 to 190 nm.

Additional compounds of the liquid formulation according to the present invention

Suitably, the liquid formulations of the present invention (further) contain one or more additional compounds selected from the group consisting of water-soluble antioxidants (compounds e)), fat-soluble antioxidants (compounds f)) and MCT (middle chain triglycerides) (compound g)).

Compound e): Water-soluble antioxidants

Suitable water-soluble antioxidants are known to the person skilled in the art. Preferably water-soluble antioxidants are used that are approved for their application in food and beverages.

Preferred water-soluble antioxidants are selected from the group consisting of citric acid, citric acid salts, ascorbic acid, ascorbic acid salts (preferably sodium ascorbate), as well as any mixture thereof.

Preferred water-soluble antioxidants are ascorbic acid, sodium ascorbate and citric acid.

Preferably the total amount of the water-soluble antioxidants in the liquid formulation according to the present invention is in the range of from 0.1 to 4.0 weight-%, more preferably it is in the range of from 0.1 to 2.0 weight-%, based on the total weight of the liquid formulation.

Compound f): Fat-soluble antioxidants

Suitable fat-soluble antioxidants are known to the person skilled in the art. Preferably fat-soluble antioxidants are used that are approved for their application in food and beverages.

Preferred fat-soluble antioxidants are selected from the group consisting of tocopherols, e.g. dl-$\alpha$-tocopherol (i.e. synthetic tocopherol), d-$\alpha$-tocopherol (i.e. natural tocopherol), $\beta$- or $\gamma$-tocopherol, or a mixture of two or more of these.

The most preferred fat-soluble antioxidant is dl-$\alpha$-tocopherol.

Preferably the total amount of the fat-soluble antioxidants in the liquid formulation according to the present invention is in the range of from 0 to 1.5 weight-%, more preferably it is in the range of from 0.01 to 1.0 weight-%, most preferably it is in the range of from 0.1 to 0.5 weight-%, based on the total weight of the liquid formulation.

Compound g)

A preferred liquid formulation according to the present invention does contain MCT (middle chain triglycerides), preferably in an amount in the range of from 0 to 5.0 weight-%, more preferably in an amount in the range of from 0.01 to 1.0 weight-%, most preferably in an amount in the range of from 0.5 to 1.0 weight-%, based on the total weight of the liquid formulation

Another preferred liquid formulation according to the present invention does contain only a minor amount of oil. The term "oil" in the context of the present invention does not encompass MCT.

Preferably the liquid formulation according to the present invention does contain only an amount of oil of at most 3 weight-%, more preferably an amount of oil of at most 2 weight-%, even more preferably an amount of oil of at most 1 weight-%, based on the total weight of the liquid formulation. In the most preferred embodiment of the present invention the liquid formulation does not contain any oil except MCT.

The term "oil" in the context of the present invention encompasses glycerol and any triglyceride such as vegetable oils or fats like corn oil, sunflower oil, soybean oil, safflower oil, rapeseed oil, peanut oil, palm oil, palm kernel oil, cotton seed oil, olive oil or coconut oil except that the term "oil" does not encompass MCT.

The oils can be from any origin. They can be natural, modified or synthetic. If the oils are natural they can be plant or animal oils. The term "oil" in the context of the present invention thus also encompasses canola oil, sesame oil, hazelnut oil, almond oil, cashew oil, macadamia oil, mongongo nut oil, pracaxi oil, pecan oil, pine nut oil, pistachio oil, sacha Inchi (Plukenetia volubilis) oil, walnut oil or polyunsaturated fatty acids (= "PUFAs") (for example arachidonic acid, eicosapentaenoic acid, docosahexaenoic acid and $\gamma$-linolenic acid) as well as the triglycerides of PUFAs and the esters of PUFAs, e.g. the ethyl esters of PUFAs.

The clear, liquid formulations according to the present invention are used for the enrichment, fortification and/or coloration of beverages; said use being a further aspect of the invention. Moreover, the invention is related to beverages

containing such clear, liquid formulations. Especially suitable beverages are soft drinks. Such soft drinks have in general a pH in the range of 2.5 to 5.0, preferably a pH in the range of 3.0 to 3.5.

Process for the manufacture of the compositions according to the invention

The present invention is further related to a process for the manufacture of a clear liquid formulation according to the present invention comprising the following steps:

i) forming a solution of the carotenoid a) in an organic solvent, optionally adding a fat-soluble antioxidant f) and/or MCT (compound g));
ii) dissolving the modified food starch b) and the saccharide c) and optionally the water-soluble antioxidant e) in water to obtain a matrix;
iii) emulsifying the solution obtained in step i) into the matrix obtained in step ii) to obtain an emulsion,
iv) removing the organic solvent from the emulsion obtained in step iii).

The steps are described in more detail below.

Step i)

An oil can also be added, but it is preferred not to add one, except MCT. If it is, however, added, the amount is chosen so that the final amount of the oil in the resulting liquid formulation after having performed all steps is as described above.

The same applies for the other compounds: the amounts of the carotenoid a), the fat-soluble antioxidant f) and the MCT are chosen so that the final amounts of these compounds in the resulting liquid formulation after having performed all steps is as described above.

The amount of the solvent and the dissolution temperature are chosen so as to dissolve the carotenoid a), the fat-soluble antioxidant f), the MCT, if present, and the oil, if present, completely. Usually it is necessary to heat up the suspension obtained when mixing all compounds present in this step to get a solution. Preferably the temperature to which the suspension is heated up is in the range of from 40 to 90°C, more preferably that temperature is in the range of from 40 to 86°C. After having obtained the solution it is usually kept at the temperature it was before heated up to.

Step ii)

Preferably this step is performed at a temperature in the range of 50 to 70°C, more preferably at a temperature in the range of 55°C to 67°C, even more preferably at a temperature of around 60°C.

The matrix obtained after having performed step ii) is then preferably kept at a temperature in the range of 25 to 65°C, more preferably at a temperature in the range of 29°C to 66°C, even more preferably at a temperature in the range of 29 to 56°C. Depending on the temperature step ii) has been performed it may be necessary to cool the matrix down to such a temperature or to heat it up to such a temperature. In most cases the temperature at which step ii) is performed and the temperature at which the matrix is kept are chosen in such a way so that a cooling down step is necessary.

Step iii)

Preferably this step is performed at a mixing temperature in the range of 25 to 100°C, more preferably at a mixing temperature in the range of 30 to 80°C, even more preferably at a mixing temperature in the range of 35°C to 75°C to obtain an emulsion.

The emulsification can be achieved by using a rotor-stator device or a high pressure homogenizer or both. Other devices known to the person skilled in the art may also be used.

If rotor-stator device and/or a high pressure homogenizer is used, a pressure drop in the range of 100 to 1000 bar, more preferably in the range of 150 to 300 bar, is preferably applied.

Step iv)

The organic solvent may e.g. be removed by using a thin film evaporator cascade (preferred). Other methods known to the person skilled in the art are also applicable.

The resulting clear liquid formulations after having performed steps i) to iv) are advantageously used as such (preferred option). They can, however, also be dried by any method known to the person skilled in the art, e.g. by spray-drying, spray-drying in combination with fluidised bed granulation or by a powder-catch technique, whereby the sprayed emulsion droplets are caught in a bed of an absorbent, such as starch, and subsequently dried. These dried forms (powders) can then also be added to beverages, especially to soft drinks.

Other aspects of the invention are beverages containing a liquid formulation as described above.

Beverages wherein the liquid formulations of the present invention can be used as a colorant or a functional ingredient can be carbonated beverages e.g., flavoured seltzer waters, soft drinks or mineral drinks, as well as non-carbonated beverages e.g. flavoured waters, fruit juices, fruit punches and concentrated forms of these beverages. They may be based on natural fruit or vegetable juices or on artificial flavours. Also included are alcoholic beverages. Besides, sugar containing beverages diet beverages with non-caloric and artificial sweeteners are also included. Especially preferred are soft drinks, preferably with a pH in the range of 2.5 to 5.0, more preferably with a pH in the range of 3.0 to 3.5.

The final concentration of the carotenoid, especially of β-carotene and 8'-apo-β-carotenal, which is added via the liquid formulations of the present invention to beverages may be from 0.1 to 50 ppm, particularly from 1 to 30 ppm, more preferably from 3 to 20 ppm, based on the total weight of the beverage and depending on the particular beverage to be coloured or fortified and the intended grade of coloration or fortification.

For coloration or fortification of a beverage a liquid formulation of this invention can be used according to methods per se known for the application of emulsions or suspensions.

Advantageously the beverages containing the liquid formulations according to the present invention are also clear **and**/or color stable, preferably they are clear and color-stable. "Color-stable" in the context of the present invention means that the color difference DE* between the initial color and the color after a storage time of 3 months should be lower than 10 (DE* < 10). A DE* < 10 means that the color difference cannot be seen by naked eyes, i.e. without the use of an apparatus such as a colorimeter.

The present invention relates to clear, stable, liquid carotenoid formulations, which - when used in a liquid, especially in a beverage such as a soft drink, allows to obtain transparent liquids (even after pasteurization and also at low pH), i.e. clear beverages.

The following non limiting examples illustrate the invention further.

## Examples

Examples 1-16: Manufacture of clear liquid formulations according to the present invention

The examples were carried out according to the following general procedure with the amounts of compounds (carotenoid, modified food starch, saccharide, antioxidants, water) and the process parameters as given in detail in Table 1-1 and Table 1-2. As solvent an organic solvent selected from the group consisting of dimethyl carbonate, ethyl formate, ethyl acetate, isopropyl acetate, methyl tert-butyl ether and methylene chloride was used. The amount of the solvent and the dissolution temperature were chosen so as to dissolve the carotenoid and the fat-soluble antioxidant and the MCT, if present, completely.

The modified food starch used was centrifuged before use.

General procedure

The amount of the carotenoid and the fat-soluble antioxidant as given in Table 1-1 or Table 1-2 are dispersed in the amount of organic solvent as given in Table 1-1 or Table 1-2, respectively.

The matrix phase consisting of the modified food starch (= OSA starch), the saccharide and the water-soluble antioxidant as given in Table 1-1/Table 1-2 is dissolved in the amount of water as given in Table 1-1/Table 1-2 at 60°C. After the dissolving, the matrix is cooled down to the temperature as given in Table 1-1/Table 1-2.

The suspension is heated up to the temperature as given in Table 1-1/Table 1-2 to dissolve the whole amount of the carotenoid. Then this solution is held at the temperature as given in Table 1-1/Table 1-2 in a holding vessel. The emulsification process consists of two steps and is carried out under pressure.

Both phases are continuously fed to a rotor stator device where the solution is emulsified into the matrix phase. The rotation speed is 5000 rpm (revolutions per minute) and the mixing temperature is as given in Table 1-1/Table 1-2. The next step entails a second micronisation step and consists of a sapphire orifice. The orifice diameter is as given in Table 1-1/Table 1-2 and the applied pressure drop over the orifice is as given in Table 1-1/Table 1-2 at the temperature as given in Table 1-1/Table 1-2. Over this orifice 10 theoretical numbers of passages are applied.

Then the organic solvent is removed from the emulsion by using a thin film evaporator cascade.

The results of the examples are summarized below. The particle size of the inner phase was measured by Photo Colleration Spectroscopy (Beckman Coulter N4 Plus Submicron Particle Sizer).

Example 1

The final product has a 8'-apo-β-carotenal content of 2.8%, E1/1 = 1625, a particle size of the inner phase of 198 nm and a turbidity of 26.7 NTU (measured in water with a concentration of the 8'-apo-β-carotenal of 10 ppm) and 25.6 NTU (measured in a soft drink as prepared according to the instruction below).

Example 2

The final product has a 8'-apo-β-carotenal content of 1.9%, E1/1 = 1460, a particle size of the inner phase of 159 nm and a turbidity of 10.9 NTU (measured in water with a concentration of the 8'-apo-β-carotenal of 10 ppm) 11.0 NTU (measured in a soft drink as prepared according to the instruction below).

Example 3

The final product has a 8'-apo-β-carotenal content of 1.7%, E1/1 = 1531, a particle size of the inner phase of 155 nm and a turbidity of 7.6 NTU (measured in water with a concentration of the 8'-apo-β-carotenal of 10 ppm) and 8.4 NTU (measured in a soft drink as prepared according to the instruction below).

Example 4

The final product has a 8'-apo-β-carotenal content of 3.9%, E1/1 = 1587, a particle size of the inner phase of 158 nm and a turbidity of 10.9 NTU (measured in water with a concentration of the 8'-apo-β-carotenal of 10 ppm) and 12.8 NTU (measured in a soft drink as prepared according to the instruction below).

Example 5

The final product has a β-carotene content of 1.9%, E1/1 = 1513, a particle size of the inner phase of 118 nm and a turbidity of 11.9 NTU (measured in water with a concentration of the β-carotene of 10 ppm) and 13.0 NTU (measured in a soft drink as prepared according to the instruction below).

Example 6

In this example also a lipophilic liquid, i.e. 18 g of MCT (middle chain triglycerides), was added.

The final product has a β-carotene content of 2.2%, E1/1 = 1473, a particle size of the inner phase of 146 nm and a turbidity of 14.9 NTU (measured in water with a concentration of the β-carotene of 10 ppm) and 15.9 NTU (measured in a soft drink as prepared according to the instruction below).

Example 7

In this example no fat-soluble antioxidant was used, but as auxiliary agent 4 g of MCT (middle chain triglycerides), a lipophilic liquid.

The final product has a β-carotene content of 1.9%, E1/1 = 1423, a particle size of the inner phase of 136 nm and a turbidity of 22.7 NTU (measured in water with a concentration of the β-carotene of 10 ppm) and 23.9 NTU (measured in a soft drink as prepared according to the instruction below).

Example 8

The final product has a 8'-apo-β-carotenal content of 2.8%, E1/1 = 1371, a particle size of the inner phase of 183 nm and a turbidity of 12.1 NTU (measured in water with a concentration of the 8'-apo-β-carotenal of 10 ppm) and 13.4 NTU (measured in a soft drink as prepared according to the instruction below).

Example 9

The final product has a 8'-apo-β-carotenal content of 2.1%, E1/1 = 1519, a particle size of the inner phase of 160 nm and a turbidity of 9.9 NTU (measured in water with a concentration of the 8'-apo-β-carotenal of 10 ppm) and 7.5 NTU (measured in a soft drink as prepared according to the instruction below).

Example 10

The final product has a β-carotene content of 2.4%, E1/1 = 1470, a particle size of the inner phase of 138 nm and a turbidity of 8.1 NTU (measured in water with a concentration of the β-carotene of 10 ppm) and 9.0 NTU (measured in a soft drink as prepared according to the instruction below).

Example 11

The final product has a β-carotene content of 2.5%, E1/1 = 1322, a particle size of the inner phase of 207 nm and a turbidity of 19.4 NTU (measured in water with a concentration of the β-carotene of 10 ppm) and 18.8 NTU (measured in a soft drink as prepared according to the instruction below).

Example 12

The final product has a β-carotene content of 2.0%, E1/1 = 1288, a particle size of the inner phase of 193 nm and a turbidity of 11.1 NTU (measured in water with a concentration of the β-carotene of 10 ppm) and 11.7 NTU (measured in a soft drink as prepared according to the instruction below).

Example 13

The final product has a β-carotene content of 2.1%, E1/1 = 1326, a particle size of the inner phase of 184 nm and a turbidity of 10.3 NTU (measured in water with a concentration of the β-carotene of 10 ppm) and 10.5 NTU (measured in a soft drink as prepared according to the instruction below).

Example 14

The final product has a 8'-apo-β-carotenal content of 2.1%, E1/1 = 1446, a particle size of the inner phase of 183 nm and a turbidity of 15.1 NTU (measured in water with a concentration of the 8'-apo-β-carotenal of 10 ppm) and 17.7 NTU (measured in a soft drink as prepared according to the instruction below).

Example 15

In this example no fat-soluble antioxidant was used, but as auxiliary agent 5 g of MCT (middle chain triglycerides), a lipophilic liquid.

The final product has a 8'-apo-β-carotenal content of 3.0%, E1/1 = 1505, a particle size of the inner phase of 157 nm and a turbidity of 16.4 NTU (measured in water with a concentration of the 8'-apo-β-carotenal of 10 ppm) and 10.4 NTU (measured in a soft drink as prepared according to the instruction below).

Example 16

The final product has a β-carotene content of 2.1%, E1/1 = 1314, a particle size of the inner phase of 145 nm and a turbidity of 34.1 NTU (measured in water with a concentration of the β-carotene of 10 ppm) and 32.8 NTU (measured in a soft drink as prepared according to the instruction below).

Application trials with the liquid formulations according to examples 1-7

The liquid formulations according to examples 1-7 have been applied in soft drinks with a concentration of the carotenoid, i.e. 8'-apo-β-carotenal or β-carotene, of 10 ppm. The objective of these trials was to evaluate the performance of these samples for their application in clear beverages. For clear beverages, the liquid formulation should provide very low turbidity values (as low as possible), and the turbidity has to be stable over storage time. Furthermore, the liquid formulation has to provide a good color stability and a good performance of appearance (no ringing, absence of particles on the surface and no sedimentation).

Application trials with the liquid formulations according to examples 8-16

The liquid formulations according to examples 8-16 have also been applied in soft drinks with a concentration of the carotenoid, i.e. 8'-apo-β-carotenal or β-carotene, of 10 ppm. Here, however, only the initial turbidity was measured. The results are as given above.

Preparation of the soft drinks

The soft drinks had the following composition:

| | Ingredient | Amount of ingredient |
|---|---|---|
| 1 | Potassium sorbate | 0.2 g |
| 2 | Sugar syrup (64° Brix) | 156.2 g |
| | Ascorbic acid | 0.2 g |
| | Aqueous 50-weight-% citric acid | 5.0 g |
| | Apricot flavor (water-soluble, Givaudan 78848-56) | 0.2 g |
| | Stock solution * | 10 g (i.e. 10 ppm) |
| 3 | Water | Filled up so that a total amount of the soft drink of 1000 ml results |
| | Total amount | 1000 ml |

* From each liquid formulation according to examples 1 to 7 and 8 to 16 a stock solution was prepared, whereby the liquid formulation was diluted with water so that the stock solution had a concentration of the carotenoid (8'-apo-β-carotenal or β-carotene) of 0.1 weight-% (= 10 ppm).

The soft drinks were prepared as follows:
Potassium sorbate 1) was dissolved in water, the other ingredients 2) were added one after the other while the mixture was gently stirred. Then the resulting soft drink syrup was diluted with drink water in such an amount to result in 1000 ml of the soft drink. The pH of the soft drinks was in the range of 3.0 to 3.5.
The soft drinks were then filled in glass bottles and the bottles sealed with a metallic cap. Some of these bottles were pasteurized and some not. The bottles were stored at room temperature (temperature in the range of 18 to 27°C) and under light exposure. Color and turbidity measurements were performed directly after beverage preparation (time = 0), as well as after a storage time of 2 weeks, 30 days, 60 days and 90 days.

Pasteurization

Pasteurization of the soft drinks was conducted in a water bath. A reference bottle containing water and a thermometer was used for the control of the temperature during pasteurization. The bottles (glass bottles/200 ml) were placed in a hot water bath with a temperature of 85°C. When the temperature in the bottle has reached 80°C the bottles remained in the water bath for 1 additional minute. After the pasteurization the bottles were quickly cooled down (using cold water) to room temperature.

**Color measurements**

Color measurements for the application in food are performed with a colorimeter (Hunter Lab Ultra Scan Pro) which can other than a spectrophotometer express color values according to the psychophysical perception of color by human eye.
Color measurements are carried out after CIE guidelines (Commission International d'Eclairage). Values can be expressed either as planar coordinates L*a*b* with L* being the measuring value for lightness, with a*being the value on the red-green-axis and with b* being the value on the yellow-blue-axis.
Instrument settings:

- Color scale : CIE L*a*b* / L*C*h*
- Light source definition: D65 daylight equivalent
- Geometry : Diffuse / 8°
- Wavelengths: scan 350 to 1050 nm in 5 nm optical resolution
- Sample measurement area diameter: 19 mm (large)
- Calibration mode: Transmission/ white tile

The color difference DE* is calculated using the following equation:

$$DE^* = \sqrt{(\Delta L^*)^2 + (\Delta a^*)^2 + (\Delta b^*)^2}$$

whereby L = lightness, a = red value, and b = yellow value

$\Delta L^* = L_x^* - L_0^*$; 0 = initial value; x = time of measuring
$\Delta a^* = a_x^* - a_0^*$; 0 = initial value; x = time of measuring
$\Delta b^* = b_x^* - b_0^*$; 0 = initial value; x = time of measuring

For a good color stability, DE* should be lower than 10 (DE* < 10); this means that the color difference cannot be seen by naked eyes, i.e. without the use of an apparatus such as a colorimeter.

**Turbidity measurements**

Suspended solids (or particles) are responsible for the turbid appearance of beverages containing juice. This turbid appearance can be evaluated by turbidity measurements. Turbidity depends on the light-scattering properties of such particles: their size, their shape and their refractive index.
In this work turbidity measurements were conducted using a Turbidimeter (Hach 2100N IS®, USA) and turbidity values were given in NTU (nephelometric turbidity units). Neophelometer measures the light scattered by a sample in 90° from the incident light path (s. Fig. 1).
Fig. 1 illustrates the principle of the nephelometric turbidity measurement Instrument settings: Light source: 860 ± 10 nm LED

Results concerning the soft drinks containing 8'-apo-β-carotenal (liquid formulations according to examples 1-4)

a) Color difference

The results concerning the measurement of the color difference during storage are shown in Fig. 2 and 3.
Fig. 2 shows the color difference (DE*) in non-pasteurized soft drinks during a storage time of up to 3 months.

x-axis: Storage time in days; y-axis: Color difference (DE*) (dimensionless);
1 = soft drink containing a liquid formulation according to example 1;
2 = soft drink containing a liquid formulation according to example 2;
3 = soft drink containing a liquid formulation according to example 3;
4 = soft drink containing a liquid formulation according to example 4.

All samples showed very good (DE* < 10) and similar color stability.
Fig. 3 shows the color difference (DE*) in pasteurized soft drinks during a storage time of up to 3 months.

x-axis: Storage time in days; y-axis: Color difference (DE*) (dimensionless);
1 = soft drink containing a liquid formulation according to example 1;
2 = soft drink containing a liquid formulation according to example 2;
3 = soft drink containing a liquid formulation according to example 3;
4 = soft drink containing a liquid formulation according to example 4.

All samples showed very good (DE* < 10) and similar color stability.

b) Turbidity

The results concerning the measurement of the turbidity during storage are shown in Fig. 4 and 5.
Fig. 4 shows the turbidity of the non-pasteurized soft drinks during a storage time of up to 3 months.

x-axis: storage time in days; y-axis: turbidity in NTU;
1 = soft drink containing a liquid formulation according to example 1;
2 = soft drink containing a liquid formulation according to example 2;

3 = soft drink containing a liquid formulation according to example 3;
4 = soft drink containing a liquid formulation according to example 4.

Turbidity increased slightly with time, but in an acceptable range for clear beverages.

Fig. 5 shows the turbidity of the pasteurized soft drinks during a storage time of up to 3 months.

x-axis: storage time in days; y-axis: turbidity in NTU;
1 = soft drink containing a liquid formulation according to example 1;
2 = soft drink containing a liquid formulation according to example 2;
3 = soft drink containing a liquid formulation according to example 3;
4 = soft drink containing a liquid formulation according to example 4.

Turbidity increased slightly with time, but in an acceptable range for clear beverages.

c) Physical appearance

After 3 months of storage the non-pasteurized and pasteurized soft drinks were evaluated visually concerning their physical appearance. Hereby the samples were examined visually whether they show a ring in the bottle neck, whether they show particles on the surface and whether they show white sediments. The following schedule of notes was applied:

Ring in bottle neck:

6 = no ring
5 = hardly noticeable ring
4 = recognizable ring
3 = clear fine ring recognizable
2 = strong ring recognizable
1 = broad ring recognizable

Particles on surface:

6 = no particles

5 = 1 to 10 particles

4 = more than 10 particles

3 = not countable anymore

2 = half of the surface covered

1 = more than half of the surface covered

White sediment:

6 = no sediment

5 = slight matt glimmer

4 = fine matt sediment

3 = matt sediment

2 = strong matt sediment

1 = very strong matt sediment

For a good performance, scores should be ≥ 3.

Tab. 2 shows the results obtained for the appearance evaluation of non-pasteurized soft drinks.

| Soft drink containing a liquid formulation according to example | Ring in bottle neck | Particles on the surface | White sediment |
|---|---|---|---|
| 1 | 5 | 5 | 5 |
| 2 | 5 | 6 | 5 |
| 3 | 5 | 6 | 4 |
| 4 | 5 | 6 | 5 |

All samples showed very good performance with respect to their appearance attributes.

Tab. 3 shows the results obtained for the appearance evaluation of pasteurized soft drinks.

| Soft drink containing a liquid formulation according to example | Ring in bottle neck | Particles on the surface | White sediment |
|---|---|---|---|
| 1 | 4 | 6 | 5 |
| 2 | 5 | 6 | 5 |
| 3 | 5 | 6 | 5 |
| 4 | 6 | 6 | 5 |

Also in pasteurized drinks, all samples showed very good performance with respect to their appearance attributes.

Results concerning the soft drinks containing β-carotene (liquid formulations according to examples 5-7)

a) Color difference

The results concerning the measurement of the color difference during storage are shown in Fig. 6 and 7.

Fig. 6 shows the color difference (DE*) in non-pasteurized soft drinks during a storage time of up to 3 months.

x-axis: Storage time in days; y-axis: Color difference (DE*) (dimensionless);
5 = soft drink containing a liquid formulation according to example 5;
6 = soft drink containing a liquid formulation according to example 6;
7 = soft drink containing a liquid formulation according to example 7;

All samples showed a very good (DE* < 10) color stability.

Fig. 7 shows the color difference (DE*) in pasteurized soft drinks during a storage time of up to 3 months.

x-axis: Storage time in days; y-axis: Color difference (DE*) (dimensionless);
5 = soft drink containing a liquid formulation according to example 5;
6 = soft drink containing a liquid formulation according to example 6;
7 = soft drink containing a liquid formulation according to example 7;

All samples showed a very good (DE* < 10) color stability.

b) Turbidity

The results concerning the measurement of the turbidity during storage are shown in Fig. 8 and 9.

Fig. 8 shows the turbidity of the non-pasteurized soft drinks during a storage time of up to 3 months.

x-axis: storage time in days; y-axis: turbidity in NTU;
5 = soft drink containing a liquid formulation according to example 5;
6 = soft drink containing a liquid formulation according to example 6;
7 = soft drink containing a liquid formulation according to example 7;

Turbidity increased slightly with time, but in an acceptable range for clear beverages.

Fig. 9 shows the turbidity of the pasteurized soft drinks during a storage time of up to 3 months.

x-axis: storage time in days; y-axis: turbidity in NTU;
5 = soft drink containing a liquid formulation according to example 5;
6 = soft drink containing a liquid formulation according to example 6;
7 = soft drink containing a liquid formulation according to example 7;

Turbidity increased slightly with time, but in an acceptable range for clear beverages.

c) Physical appearance

After 3 months of storage the non-pasteurized and pasteurized soft drinks were evaluated visually concerning their physical appearance. Hereby the samples were examined visually whether they show a ring in the bottle neck, whether they show particles on the surface and whether they show white sediments. The following schedule of notes was applied:

Ring in bottle neck:

6 = no ring
5 = hardly noticeable ring
4 = recognizable ring
3 = clear fine ring recognizable
2 = strong ring recognizable
1 = broad ring recognizable

Particles on surface:

6 = no particles
5 = 1 to 10 particles
4 = more than 10 particles
3 = not countable anymore
2 = half of the surface covered
1 = more than half of the surface covered

White sediment:

6 = no sediment
5 = slight matt glimmer
4 = fine matt sediment
3 = matt sediment
2 = strong matt sediment

1 = very strong matt sediment

For a good performance, scores should be ≥ 3.

Table 4 shows the results obtained for the appearance evaluation of non-pasteurized soft drinks.

| **Soft drink containing a liquid formulation according** to **example** | **Ring in bottle neck** | **Particles on the surface** | **White sediment** |
|---|---|---|---|
| **5** | 6 | 6 | 5 |
| **6** | 5 | 6 | 4 |
| **7** | 4 | 6 | 4 |

All samples showed very good performance with respect to their appearance attributes.

Table 5 shows the results obtained for the appearance evaluation of pasteurized soft drinks.

| **Soft drink containing a liquid formulation according to example** | **Ring in bottle neck** | **Particles on the surface** | **White sediment** |
|---|---|---|---|
| **5** | 6 | 6 | 6 |
| **6** | 6 | 6 | 6 |
| **7** | 4 | 6 | 6 |

All samples showed very good performance with respect to their appearance attributes.

Table 1-1: In this table A = 8'-apo-β-carotenal, B = β-carotene, D = dl-α-tocopherol; H = HiCap 100; MCT = middle chain triglycerides; S = Sodium ascorbate.

| **Example** | **1** | **2** | **3** | **4** | **5** | **6** | **7** | **8** |
|---|---|---|---|---|---|---|---|---|
| **Carotenoid** | A | A | A | A | B | B | B | A |
| **Amount of carotenoid [g]** | 32 | 20 | 21 | 43 | 22 | 38 | 22 | 27 |
| **Fat-soluble Antioxidant** | D | D | D | D | D | D | none | D |
| **Amount of fat-soluble antioxidant [g]** | 6 | 4 | 4 | 8 | 4 | 7 | - | 5 |
| **Amount of MCT [g]** | 32 | - | - | - | - | 18 | 9 | - |
| **Modified food starch** | H | H | H | H | H | H | H | H |
| **Amount of modified food starch [g]** | 725 | 489 | 472 | 468 | 538 | 653 | 531 | 420 |
| **Saccharide and** | 71 g | 41 g of a | 40 g of a | 39 g of a | 45 g of a | 319 g of a | 44 g of a | 36 g of a |

(continued)

| Example | 1 | 2 | 3 | 4 | 5 | 6 | 7 | 8 |
|---|---|---|---|---|---|---|---|---|
| **amount** | invert sugar | glucose syrup with a DE = 47 | glucose syrup with a DE = 95 and 40 g of a glucose syrup with a DE = 47 | glucose syrup with a DE = 95 and 39 g of a glucose syrup with a DE = 47 | glucose syrup with a DE = 47 | glucose syrup with a DE = 95 | glucose syrup with a DE = 47 | glucose syrup with a DE = 95 and 36 g of a glucose syrup with a DE = 47 |
| **Water-soluble antioxidant** | none | S | S | S | S | none | S& ascorbic acid | S |
| **Amount of water-soluble antioxidant [g]** | - | 16 | 16 | 16 | 18 | - | 18 & 4 | 14 |
| **Amount of water [g]** | 796 | 1016 | 1093 | 1083 | 1117 | 523 | 1101 | 1182 |
| **Temperature to which the matrix is cooled down** | 63°C | 43°C | 46°C | 55°C | 30°C | 66°C | 29°C | 54°C |
| **Temperature to which the suspension is heated up** | 67°C | 50°C | 50°C | 76°C | 40°C | 73°C | 44°C | 56°C |
| **Temperature at which the solution is held** | 76°C | 46°C | 46°C | 72°C | 40°C | 75°C | 49°C | 55°C |
| **Mixing temperature** | 60°C | 44°C | 45°C | 54°C | 36°C | 57°C | 35°C | 58°C |
| **Orifice diameter [μm]** | 200 | 170 | 170 | 200 | 200 | 170 | 170 | 200 |
| **Applied pressure drop at temperature given** | 214 bar at 70°C | 238 bar at 54°C | 245 bar at 53°C | 197 bar at 60°C | 146 bar at 39°C | 195 bar at 72°C | 168 bar at 42°C | 249 bar at 59°C |

Table 1-2: In this table A = 8'-apo-β-carotenal, B = β-carotene, D = dl-α-tocopherol; H = HiCap 100; MCT = middle chain triglycerides; S = Sodium ascorbate.

| Example | 9 | 10 | 11 | 12 | 13 | 14 | 15 | 16 |
|---|---|---|---|---|---|---|---|---|
| **Carotenoid** | A | B | B | B | B | A | A | B |
| **Amount of carotenoid [g]** | 21 | 26 | 20 | 20 | 23 | 21 | 24 | 14 |

(continued)

| Example | 9 | 10 | 11 | 12 | 13 | 14 | 15 | 16 |
|---|---|---|---|---|---|---|---|---|
| Fat-soluble Antioxidant | none | D | D | D | D | D | none | D |
| Amount of fat-soluble antioxidant [g] | - | 5 | 4 | 4 | 5 | 4 | - | 3 |
| Amount of MCT [g] | - | - | - | - | - | - | 5 | - |
| Modified food starch | H | H | H | H | H | H | H | ClearGum Co03 |
| Amount of modified food starch [g] | 449 | 437 | 489 | 489 | 568 | 509 | 171 | 343 |
| Saccharide and amount | 39 g of a glucose syrup with a DE = 95 and 39 g of a glucose syrup with a DE = 47 | 38 g of a glucose syrup with a DE = 47 | 41 g of a glucose syrup with a DE = 47 | 41 g of maltodextrin | 48 g of fructose | 43 g of glucose syrup with a DE = 65 | 125 g of a glucose syrup with a DE = 95 and 125 g of a glucose syrup with a DE = 47 | 29 g of a glucose syrup with a DE = 47 |
| Water-soluble antioxidant | S | S | S | S | S | S | S | S |
| Amount of water-soluble antioxidant [g] | 15 | 15 | 16 | 16 | 19 | 17 | 17 | 6 |
| Amount of water [g] | 1006 | 1142 | 1016 | 1016 | 1178 | 1056 | 1317 | 880 |
| Temperature to which the matrix is cooled down | 50°C | 52°C | 44°C | 49°C | 55°C | 54°C | 56°C | 42°C |
| Temperature to which the suspension is heated up | 54°C | 69°C | 69°C | 65°C | 85°C | 48°C | 85°C | 41°C |
| Temperature at which the solution is held | 54°C | 76°C | 69°C | 73°C | 86°C | 46°C | 85°C | 44°C |

(continued)

| Example | 9 | 10 | 11 | 12 | 13 | 14 | 15 | 16 |
|---|---|---|---|---|---|---|---|---|
| **Mixing temperature** | 57°C | 75°C | 53°C | 54°C | 54°C | 44°C | 74°C | 45°C |
| **Orifice diameter [μm]** | 200 | 200 | 200 | 170 | 170 | 200 | 200 | 170 |
| **Applied pressure drop at temperature given** | 246 bar at 51 °C | 246 bar at 59°C | 229 bar at 61°C | 210 bar at 61°C | 244 bar at 62°C | 246 bar at 60°C | 250 bar at 60°C | 214 bar at 53°C |

## Claims

1. A clear liquid formulation comprising:

    a) at least one carotenoid,
    b) at least one modified food starch,
    c) at least one saccharide, and
    d) water,

    wherein the clear liquid formulation does not include at least one of the of the following:

    • Gum acacia;
    • Gum ghatti;
    • Proteins such as gelatin;
    • Plant proteins;
    • Milk proteins;
    • Ligninsulfonate;
    • Conjugates of plant gums and modified food starch;
    • Sodium lauryl sulfate and other sodium alkyl sulfates..

2. The clear liquid formulation according to claim 1, wherein the carotenoid a) is embedded in a matrix of the modified food starch b) and the saccharide c).

3. The clear liquid formulation according to claim 1, wherein the clear liquid formulation when diluted so that the concentration of the at least one carotenoid is 10 ppm has an initial turbidity of less than 30 NTU.

4. The clear liquid formulation according to claim 1 and/or 2, wherein the saccharide c) is present in an amount in the range of 0.5 to 60 weight-%, preferably in an amount in the range of 0.5 to 30 weight-%, more preferably in an amount in the range of 0.5 to 20 weight-%, even more preferably in an amount in the range of 0.5 to 10 weight-%, based on the total weight of the clear liquid formulation, **and/or** wherein the carotenoid a) is present in an amount in the range of 0.1 to 10 weight-%, preferably in an amount in the range of 0.5 to 5.0 weight-%, more preferably in an amount in the range of 0.5 to 3.0 weight-%, even more preferably in an amount in the range of 1.0 to 3.0 weight-%, based on the total weight of the clear liquid formulation, **and/or** wherein the modified food starch b) is present in an amount in the range of 20 to 60 weight-%, more preferably in an amount in the range of 30 to 50 weight-%, based on the total weight of the clear liquid formulation, **and/or** wherein the water d) is present in an amount in the range of 35 to 75 weight-%, more preferably in an amount in the range of 45 to 65 weight-%, based on the total weight of the clear liquid formulation.

5. The clear liquid formulation according to any one or more of the preceding claims, wherein the carotenoid a) is selected from the group consisting of α-carotene, β-carotene, 8'-apo-β-caroten-8'-al, 8'-apo- β-carotenoic acid esters, canthaxanthin, astaxanthin, astaxanthin (di)esters, lycopene, lutein, zeaxanthin or crocetin, and mixtures thereof,

preferably wherein the carotenoid is β-carotene or 8'-apo-β-caroten-8'-al or a mixture thereof.

**6.** A clear liquid formulation comprising:

a) 0.1 to 10 weight-% (preferably 0.5 to 5 weight-%, more preferably 0.5 to 3.0 weight-%, most preferably 1.0 to 3.0 weight-%) of at least one carotenoid, and
b) 20 to 60 weight-% (preferably 30 to 50 weight-%) of at least one modified food starch, and
c) 0.5 to 60 weight-% (preferably 0.5 to 30 weight-%, more preferably 0.5 to 20 weight-%, even more preferably 0.5 to 10 weight-%, most preferably 1.0 to 10 weight-%) of at least one saccharide, and
d) 35 to 75 weight-% (preferably 45 to 65 weight-%) of water, all amounts based on the total weight of the liquid formulation,

whereby all amounts add up to 100 weight-%.

**7.** The clear liquid formulation according to any one or more of the preceding claims further comprising e) water-soluble antioxidants, preferably selected from the group consisting of ascorbic acid, sodium ascorbate, citric acid and any mixture thereof, **and/or** wherein the total amount of the water-soluble antioxidants e) is in the range of 0.1 to 4.0 weight-%, preferably in the range of 0.1 to 2.0 weight-%, based on the total weight of the liquid formulation.

**8.** The liquid formulation according to any one or more of the preceding claims further comprising f) fat-soluble antioxidants, preferably further comprising dl-α-tocopherol, **and/or** wherein the total amount of the fat-soluble antioxidants f) is in the range of 0 to 1.5 weight-%, preferably in the range of 0.01 to 1.0 weight-%, more preferably in the range of 0.1 to 0.5 weight-%, based on the total weight of the liquid formulation.

**9.** The liquid formulation according to any one or more of the preceding claims, wherein the liquid formulation does not contain any oil except middle chain triglycerides.

**10.** The liquid formulation according to any one or more of the preceding claims further comprising g) middle chain triglycerides, preferably in an amount in the range of 0 to 5.0 weight-%, more preferably in an amount in the range of 0.01 to 1.0 weight-%, even more preferably in an amount in the range of 0.5 to 1.0 weight-%, based on the total weight of the liquid formulation.

**11.** The liquid formulation according to any one or more of claims 1 to 5 consisting only of compounds a) to d).

**12.** The liquid formulation according to claim 6 consisting only of compounds a) to e).

**13.** The liquid formulation according to claim 7 consisting only of compounds a) to f).

**14.** The liquid formulation according to any one or more of claims 8 to 9 consisting only of compounds a) to g).

**15.** The clear liquid formulation according to any one or more of the preceding claims, wherein the modified food starch b) has been centrifuged before use.

**16.** The clear liquid formulation according to any one or more of the preceding claims, wherein the particle size of the inner phase is in the range of 100 to 250 nm, preferably in the range of 110 to 210 nm, more preferably in the range of 130 to 190 nm.

**17.** The clear liquid formulation according to claim 1, wherein the clear liquid formulation does not include middle chain triglycerides.

**18.** Process for the manufacture of a clear liquid formulation according to any one or more of the preceding claims comprising the following steps:

i) forming a solution of the carotenoid a) in an organic solvent, optionally adding a fat-soluble antioxidant f) and/or MCT (compound g));
ii) dissolving the modified food starch b) and the saccharide c) and optionally the water-soluble antioxidant e) in water to obtain a matrix;
iii) emulsifying the solution obtained in step i) into the matrix obtained in step ii) to obtain an emulsion;

iv) removing the organic solvent from the emulsion obtained in step iii).

**19.** Beverage comprising at least a liquid formulation according to any one or more of the claims 1-18, preferably wherein the beverage is clear and/or color stable.

**20.** The beverage as in claim 19, wherein the clear and/or stable beverage has been pasteurized.

Fig. 1

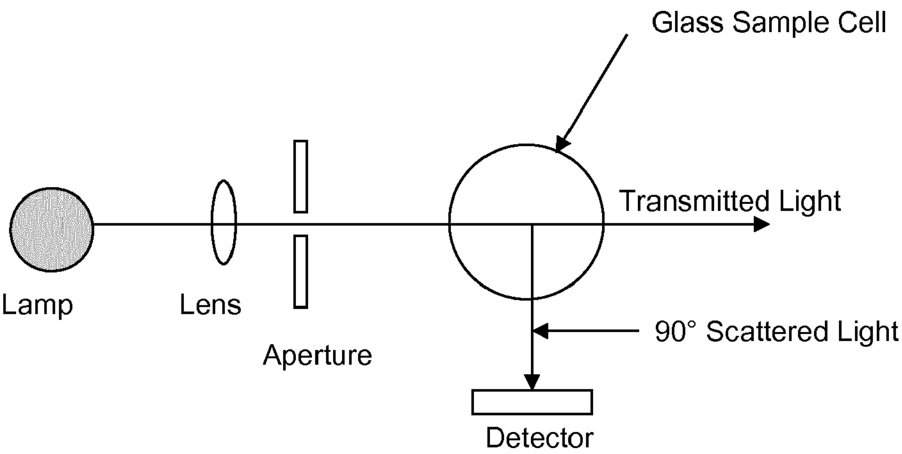
Glass Sample Cell
Transmitted Light
Lamp
Lens
Aperture
90° Scattered Light
Detector

Fig. 2

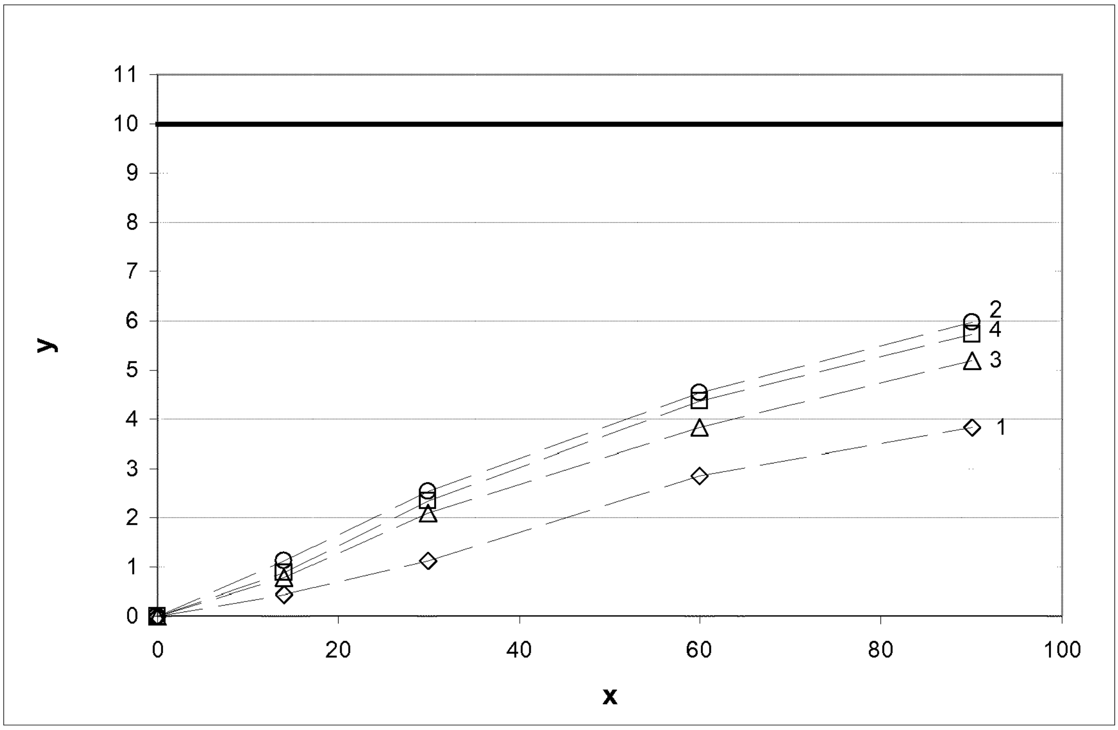

Fig. 3

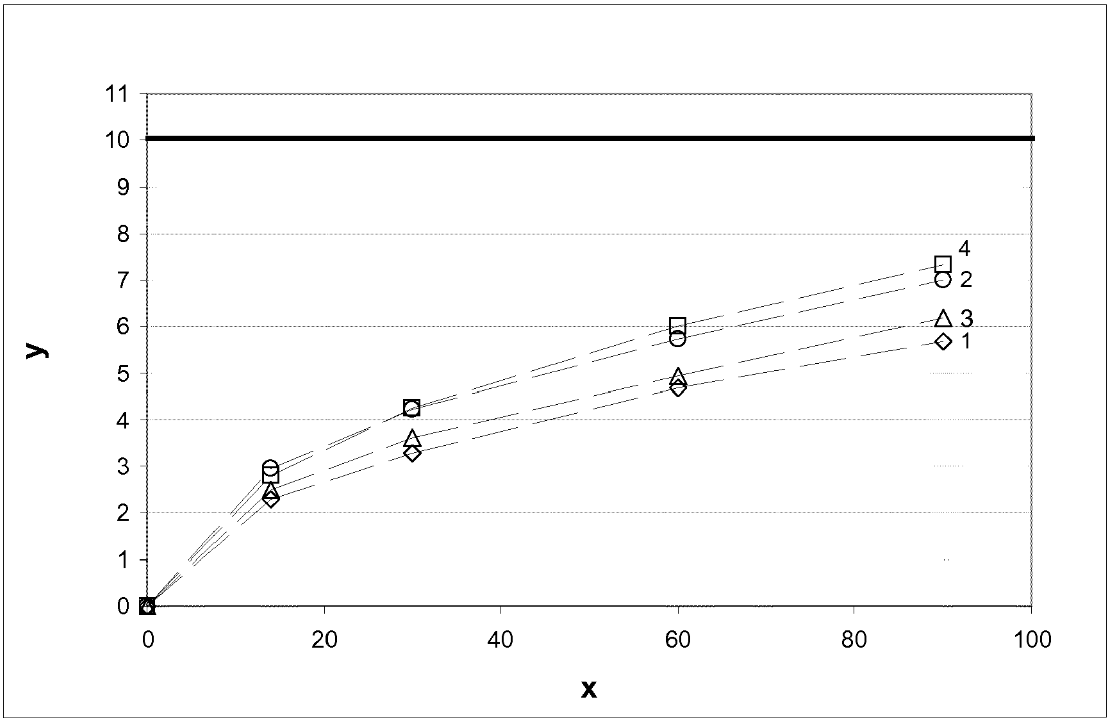

Fig. 4

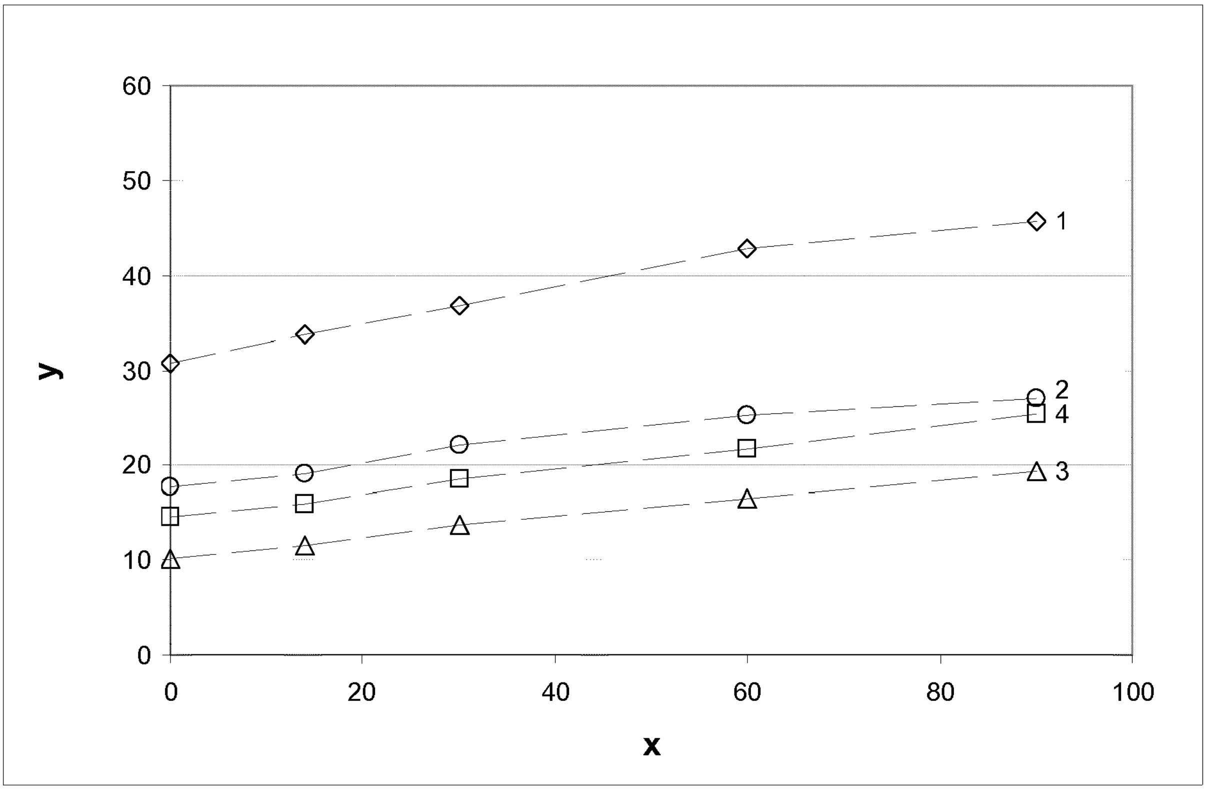

Fig. 5

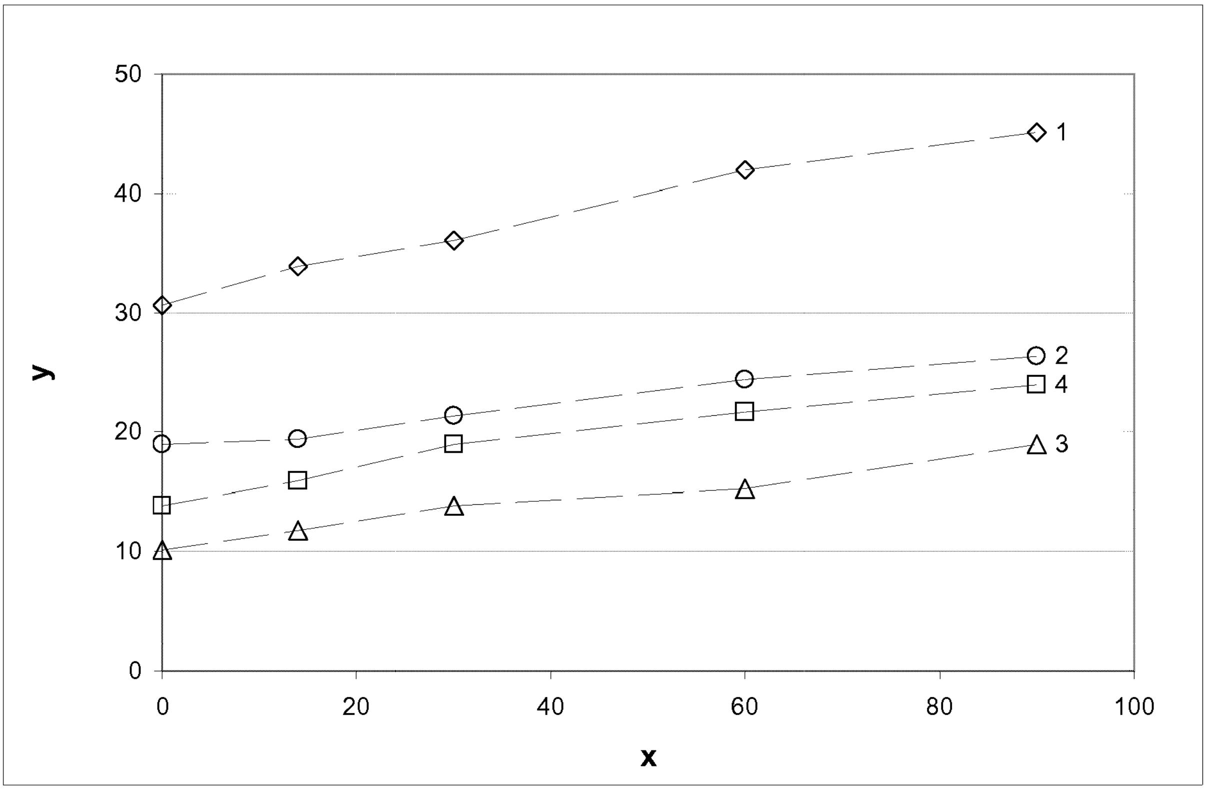

Fig. 6

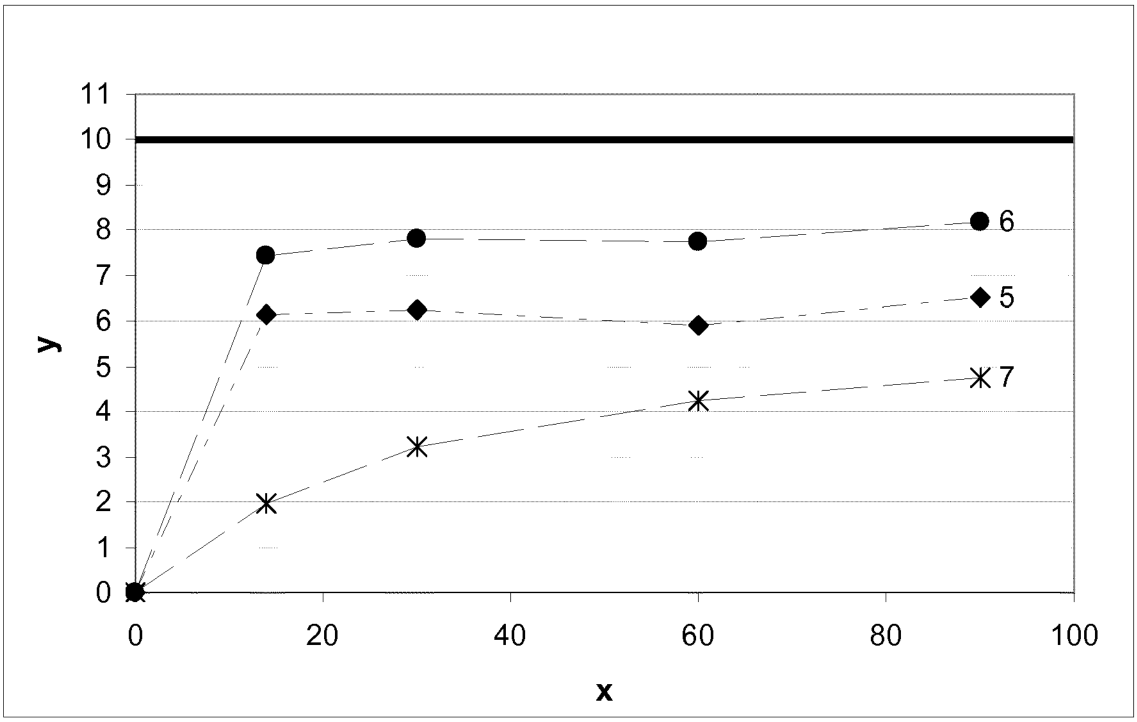

Fig. 7

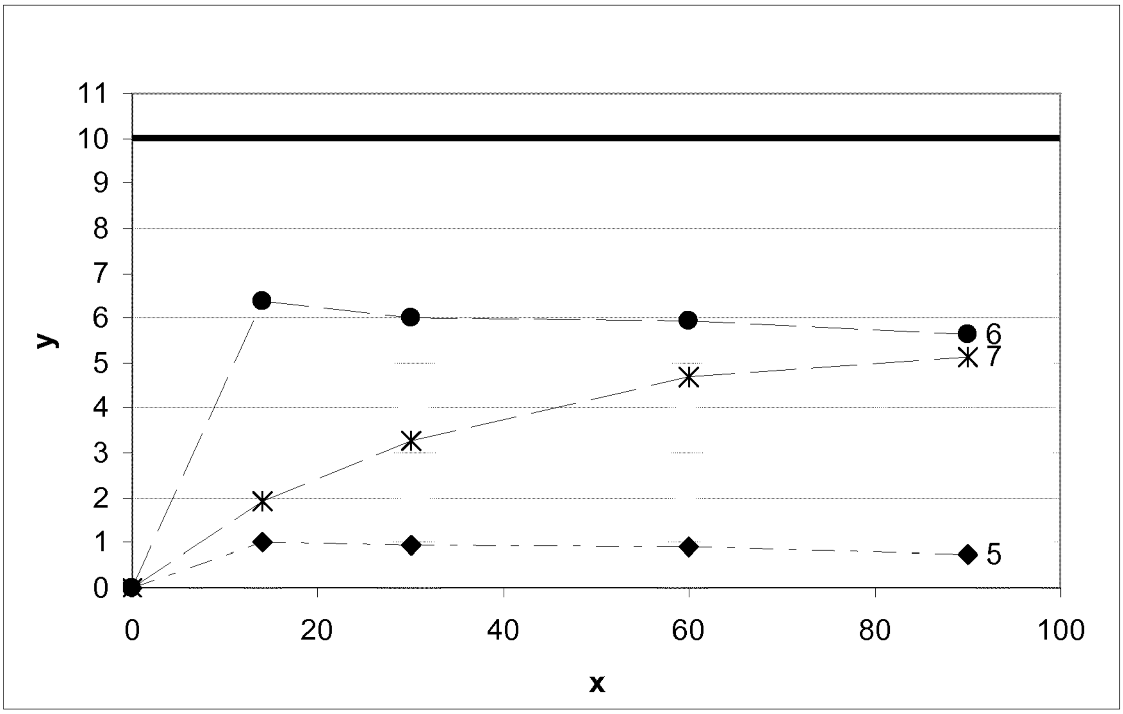

Fig. 8

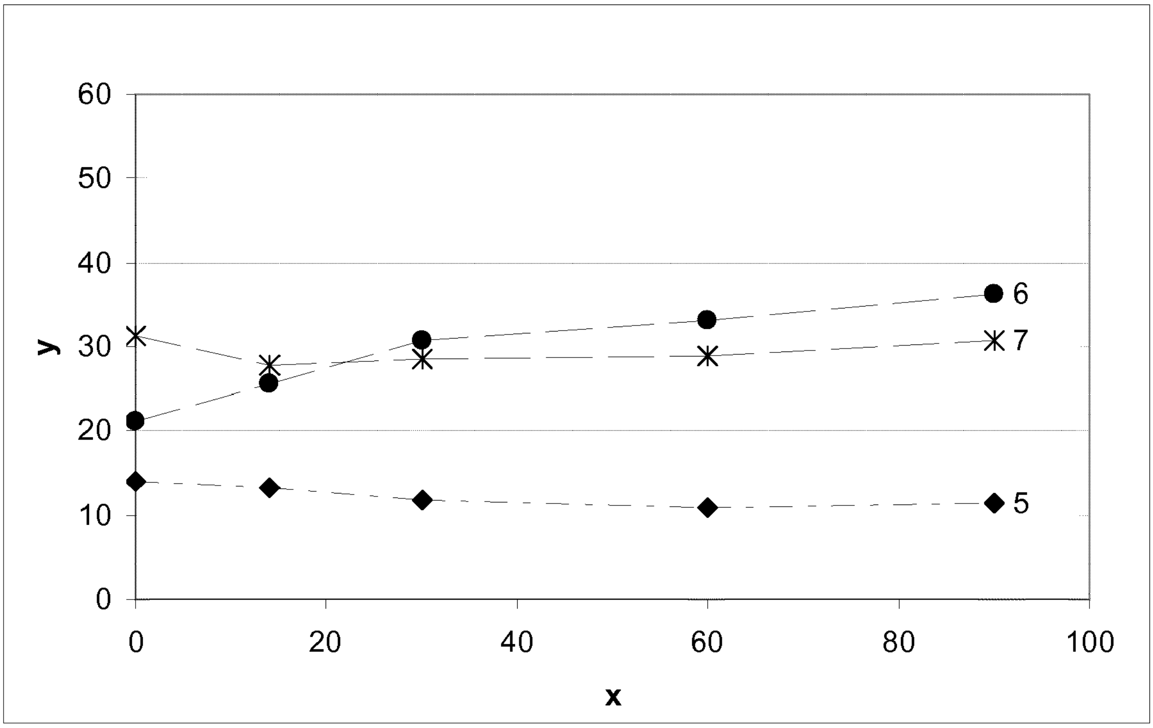

Fig. 9

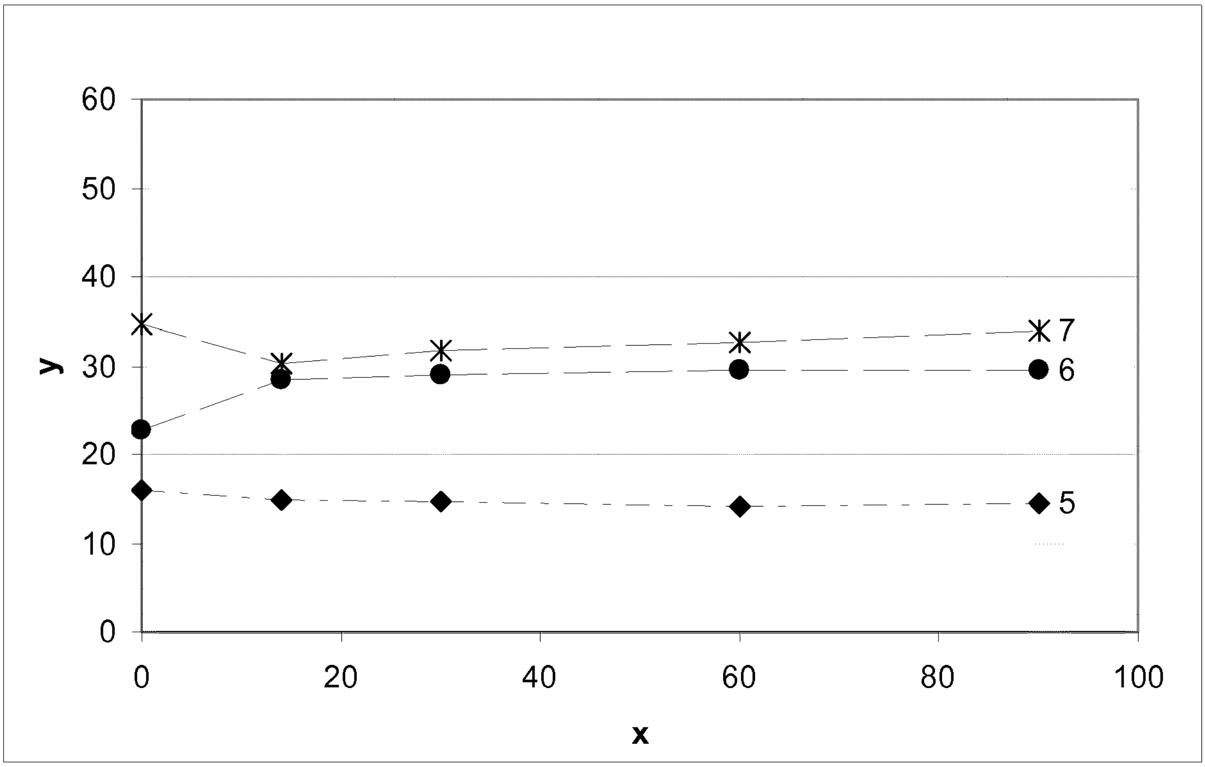

Europäisches Patentamt
European Patent Office
Office européen des brevets

# EUROPEAN SEARCH REPORT

Application Number
EP 22 18 6936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 964 479 A1 (DSM IP ASSETS BV [NL]) 3 September 2008 (2008-09-03) * paragraphs [0016], [0017], [0035]; claims 1,10; examples 1,2 * | 1-17 | INV. A23L2/58 A23L5/44 A61K31/015 A61K31/11 |
| X | WO 2008/110225 A1 (DSM IP ASSETS BV [NL]; HITZFELD ANDREA [DE]; LEUENBERGER BRUNO H [CH];) 18 September 2008 (2008-09-18) * page 3, paragraph 2-5; figures 1-7; examples 2-4,6 * * page 12, line 6 - page 13, paragraph 4 * | 1-6, 10-17 | |
| X | US 2010/028444 A1 (MATUSCHEK MARKUS [DE] ET AL) 4 February 2010 (2010-02-04) * paragraphs [0018] - [0022]; examples A3,A11,A13,A14 * | 1-20 | |
| X | WO 2007/009601 A1 (DSM IP ASSETS BV [NL]; VOELKER KARL MANFRED [DE]) 25 January 2007 (2007-01-25) * paragraphs [0010] - [0024]; claims 1-15; examples 1,2 * | 1-17 | TECHNICAL FIELDS SEARCHED (IPC) |
| X | WO 2011/039336 A1 (DSM IP ASSETS BV [NL]; VIDONI OLIVIA [FR]; HITZFELD ANDREA [DE]; LEUEN) 7 April 2011 (2011-04-07) * page 3, lines 18-25; claims 1,6,8,9,11; examples 1-7 * | 6,10-17 | A23L A61K |
| X | WO 2009/147158 A2 (DSM IP ASSETS BV [NL]; HITZFELD ANDREA [DE]; VIDONI OLIVIA [FR]; LEUEN) 10 December 2009 (2009-12-10) * page 2, lines 15-29; examples 1-4 * | 6,10-17 | |
| X | US 3 998 753 A (ANTOSHKIW THOMAS WILLIAM ET AL) 21 December 1976 (1976-12-21) * column 1, lines 50-70; claims 1-21; examples 1-4 * | 6,10-17 | |

The present search report has been drawn up for all claims

2

| Place of search | Date of completion of the search | Examiner |
|---|---|---|
| The Hague | 30 November 2022 | Tallgren, Antti |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 22 18 6936

This annex lists the patent family members relating to the patent documents cited in the above-mentioned European search report. The members are as contained in the European Patent Office EDP file on
The European Patent Office is in no way liable for these particulars which are merely given for the purpose of information.

30-11-2022

| Patent document cited in search report | Publication date | Patent family member(s) | Publication date |
|---|---|---|---|
| EP 1964479 A1 | 03-09-2008 | CN 101610683 A | 23-12-2009 |
| | | EP 1964479 A1 | 03-09-2008 |
| | | ES 2422458 T3 | 11-09-2013 |
| | | PL 1964479 T3 | 30-09-2013 |
| | | US 2010136177 A1 | 03-06-2010 |
| | | WO 2008098694 A1 | 21-08-2008 |
| WO 2008110225 A1 | 18-09-2008 | CN 101631803 A | 20-01-2010 |
| | | EP 2118146 A1 | 18-11-2009 |
| | | ES 2727933 T3 | 21-10-2019 |
| | | JP 5364894 B2 | 11-12-2013 |
| | | JP 2010521144 A | 24-06-2010 |
| | | KR 20090119976 A | 23-11-2009 |
| | | PL 2118146 T3 | 30-09-2019 |
| | | TR 201907634 T4 | 21-06-2019 |
| | | US 2010028466 A1 | 04-02-2010 |
| | | US 2012149782 A1 | 14-06-2012 |
| | | WO 2008110225 A1 | 18-09-2008 |
| US 2010028444 A1 | 04-02-2010 | AT 494799 T | 15-01-2011 |
| | | BR PI0807583 A2 | 01-07-2014 |
| | | DK 2139347 T3 | 18-04-2011 |
| | | EP 2139347 A2 | 06-01-2010 |
| | | JP 2010518829 A | 03-06-2010 |
| | | US 2010028444 A1 | 04-02-2010 |
| | | WO 2008102019 A2 | 28-08-2008 |
| WO 2007009601 A1 | 25-01-2007 | CN 101312655 A | 26-11-2008 |
| | | EP 1903887 A1 | 02-04-2008 |
| | | EP 2272380 A1 | 12-01-2011 |
| | | JP 2009501534 A | 22-01-2009 |
| | | KR 20080031418 A | 08-04-2008 |
| | | TW 200734412 A | 16-09-2007 |
| | | US 2008193539 A1 | 14-08-2008 |
| | | US 2014018442 A1 | 16-01-2014 |
| | | WO 2007009601 A1 | 25-01-2007 |
| WO 2011039336 A1 | 07-04-2011 | CN 102573513 A | 11-07-2012 |
| | | EP 2482672 A1 | 08-08-2012 |
| | | US 2012259023 A1 | 11-10-2012 |
| | | WO 2011039336 A1 | 07-04-2011 |
| WO 2009147158 A2 | 10-12-2009 | CN 102056497 A | 11-05-2011 |
| | | EP 2280611 A2 | 09-02-2011 |
| | | ES 2436167 T3 | 27-12-2013 |
| | | JP 2011521658 A | 28-07-2011 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 22 18 6936

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2022

| Patent document cited in search report | Publication date | Patent family member(s) | Publication date |
|---|---|---|---|
| | | PL 2280611 T3 | 31-01-2014 |
| | | US 2011081330 A1 | 07-04-2011 |
| | | WO 2009147158 A2 | 10-12-2009 |
| US 3998753 A | 21-12-1976 | AT 351912 B | 27-08-1979 |
| | | BE 832331 A | 12-02-1976 |
| | | DE 2534091 A1 | 26-02-1976 |
| | | FR 2281961 A1 | 12-03-1976 |
| | | GB 1502895 A | 08-03-1978 |
| | | IT 1050722 B | 20-03-1981 |
| | | JP S5141732 A | 08-04-1976 |
| | | NL 7509586 A | 17-02-1976 |
| | | US 3998753 A | 21-12-1976 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100028444 A
- WO 2008110225 A
- WO 2009147158 A
- WO 2011039336 A
- WO 2007090614 A


### Non-patent literature cited in the description


- Modified Starches: Properties and Uses. CRC Press, Inc, 1986